(19) **Europäisches Patentamt / European Patent Office / Office européen des brevets**

(11) **EP 3 251 638 B1**

(12) **EUROPÄISCHE PATENTSCHRIFT**

(45) Veröffentlichungstag und Bekanntmachung des Hinweises auf die Patenterteilung:
**01.06.2022 Patentblatt 2022/22**

(21) Anmeldenummer: **17173237.3**

(22) Anmeldetag: **18.04.2008**

(51) Internationale Patentklassifikation (IPC):
***A61F 9/008*** (2006.01)

(52) Gemeinsame Patentklassifikation (CPC):
**A61F 9/008; A61F 9/00827;** A61F 9/00831; A61F 9/00836; A61F 2009/00872; A61F 2009/0088

(54) **PLANUNGSEINRICHTUNG FÜR EINE BEHANDLUNGSVORRICHTUNG ZUR AUGENCHIRURGIE**

PLANNING DEVICE FOR A TREATMENT DEVICE FOR USE IN EYE SURGERY

DISPOSITIF DE PLANIFICATION POUR UN DISPOSITIF DE TRAITEMENT POUR LA CHIRURGIE OPHTALMOLOGIQUE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: 26.04.2007 DE 102007019814
26.04.2007 US 914179 P

(43) Veröffentlichungstag der Anmeldung:
**06.12.2017 Patentblatt 2017/49**

(62) Dokumentnummer(n) der früheren Anmeldung(en) nach Art. 76 EPÜ:
**08735334.8 / 2 136 747**

(73) Patentinhaber: **Carl Zeiss Meditec AG**
**07745 Jena (DE)**

(72) Erfinder:
• **BISCHOFF, Mark**
**07749 Jena (DE)**
• **Mühlhoff, Dirk**
**07751 Jena (DE)**
• **STOBRAWA, Gregor**
**07743 Jena (DE)**

(74) Vertreter: **Patentanwälte Geyer, Fehners & Partner mbB**
**Perhamerstrasse 31**
**80687 München (DE)**

(56) Entgegenhaltungen:
**WO-A1-2007/012924 WO-A2-2006/087180**

**Beschreibung**

Die Erfindung bezieht sich auf eine Behandlungsvorrichtung zur Refraktion korrigierenden Augenchirurgie, die mittels einer Lasereinrichtung mindestens eine Schnittfläche in der Hornhaut erzeugt.

Im Stand der Technik sind verschiedenste Behandlungsverfahren mit dem Ziel der Refraktionskorrektur am menschlichen Auge bekannt. Ziel der Operationsmethoden ist es dabei, die Hornhaut gezielt zu verändern, um so die Lichtbrechung zu beeinflussen. Hierfür werden mehrere Operationsmethoden eingesetzt.

Die WO 2007/012924 A1 befasst sich mit der Ermittlung von Informationen über das Auge vor einer laserchirurgischen Refraktionskorrektur durch Ablation.

Zur Refraktionskorrektur am verbreitetsten ist gegenwärtig die sogenannte Laser-Insitu-Keratomileusis, die auch LASIK abgekürzt wird. Bei diesem Eingriff wird zuerst eine Hornhaut-Lamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp., Irvine, USA, vertrieben wird. Nachdem die Lamelle gelöst und zur Seite geklappt wurde, ist bei der LASIK-Operation die Anwendung eines Excimer-Lasers vorgesehen, der das derart unter der Lamelle freigelegte Hornhautgewebe durch Ablation abträgt. Nachdem auf diese Art und Weise unter der Hornhautoberfläche liegendes Volumen verdampft wurde, wird die Hornhaut-Lamelle wieder auf den ursprünglichen Platz zurückgeklappt.

Die Anwendung eines Laserkeratoms zum Freilegen der Lamelle ist gegenüber einem mechanischen Messer vorteilhaft, da die Infektionsgefahr verringert und zugleich die Schnittqualität verbessert ist. Insbesondere kann die Lamelle mit sehr viel konstanterer Dicke hergestellt werden, wenn Laserstrahlung verwendet wird. Auch ist der Schnitt meist glatter, was die Gefahr für spätere optische Störungen durch diese auch nach der Operation verbleibende Grenzfläche mindert. Nachteilig bei diesem Verfahren ist allerdings, dass zwei unterschiedliche Behandlungsvorrichtungen verwendet werden müssen, zum einen nämlich das Laserkeratom zum Freilegen der Lamelle und zum anderen der das Hornhautgewebe verdampfende Laser.

Diese Nachteile sind behoben bei einem Verfahren, das jüngst durch die Carl Zeiss Meditec AG implementiert wurde und mit der Bezeichnung FLEX abgekürzt wird. Bei diesem Verfahren werden mittels eines Femtosekundenlasers in der Augenhornhaut eine Schnittgeometrie gebildet, welche in der Hornhaut ein Hornhaut-Volumen (sog. Lentikel) separiert. Dieses wird dann manuell vom Operateur entnommen. Der Vorteil dieses Verfahrens liegt zum einen darin, dass die Schnittqualität durch Anwendung des Femtosekundenlasers nochmals verbessert ist. Zum anderen ist nur noch eine Behandlungsvorrichtung erforderlich; der Excimer-Laser wird nicht mehr eingesetzt.

Bei der Erzeugung von Schnittflächen in der Hornhaut mittels Laserstrahlung wird üblicherweise die optische Strahlungswirkung dadurch ausgenutzt, dass ein optischer Durchbruch erzeugt wird. Auch ist es bekannt, einzelne Pulse, deren Energie unter einem Schwellwert für einen optischen Durchbruch liegt, derart überdeckt ins Gewebe bzw. Material einzubringen, dass auch damit eine Material- bzw. Gewebetrennung erreicht wird. Dieses Konzept der Schnitterzeugung im Hornhautgewebe erlaubt eine große Vielfalt an Schnitten.

Bei den geschilderten laserchirurgischen Operationsverfahren verbleibt als Ergebnis der Behandlung ein Schnitt in der Cornea, welcher zwar nach kurzer Zeit mit dem bloßen Auge nicht mehr sichtbar ist, aber aufgrund der Besonderheit der Augenhornhaut nie verheilt, da die Augenhornhaut in dieser Hinsicht "totes" Gewebe ist. Die Gewebeteile oberhalb eines Schnittes sind über den Schnitt nicht mehr fest mit den Gewebeteilen unterhalb des Schnittes verbunden.

Es kann aber der Bedarf von Nachbehandlungen entstehen, wenn nämlich entweder das Ergebnis des vorherigen Eingriffes hinsichtlich der Refraktionskorrektur noch nicht zufriedenstellend ist, oder wenn der vorherige Eingriff aus irgendeinem Grunde nicht ausreichend abgeschlossen werden konnte (z.B. aufgrund eines Abbruchs des Eingriffs).

Im Falle einer ungenügenden refraktiven Korrektur ist es für die Excimer-Laser basierte LASIK-Operation bekannt für die Nachbehandlung, noch einmal die Hornhaut-Lamelle abzuheben und weiteres Hornhautgewebe abzutragen.

Für das FLEX-Verfahren ist dieser Ansatz allerdings unpraktikabel, da eine Nachbehandlung dann nicht mit dem gleichen Gerät wie der vorherige Eingriff durchgeführt werden könnte, wodurch man gezwungen wäre, lediglich für den Fall der Nachbehandlung ein weiteres Gerät vorzuhalten.

Für abgebrochene laserbasierte FLEX-Operationen ist sogar überhaupt keine sinnvolle oder sichere Lösung bekannt.

Der Erfindung liegt deshalb die Aufgabe zugrunde, eine Behandlungsvorrichtung zur Refraktion korrigierenden Augenchirurgie sowie ein Verfahren zum Erzeugen von Steuerdaten für eine solche Behandlungsvorrichtung bzw. ein Verfahren zur Refraktion korrigierenden Augenchirurgie anzugeben, bei dem einfach eine Nachbehandlung ohne Ablation von Hornhautgewebe bzw. die Fortsetzung einer abgebrochenen Behandlung möglich ist.

Die Erfindung ist im Anspruch 1 definiert.

Die Behandlungsvorrichtung zur Refraktion korrigierenden Augenchirurgie weist eine Lasereinrichtung, welche mittels Laserstrahlung gemäß Steuerdaten zumindest eine Schnittfläche in der Hornhaut erzeugt, und eine Planungseinrichtung zum Erzeugen der Steuerdaten auf.

Durch eine optionale Berücksichtigung der voroperativen Schnitte, also derjenigen Schnitte, die nach einem vorherigen Eingriff in die Hornhaut schon bestehen, kann eine Nachbehandlung sowohl in Fällen, in denen noch eine verbleibende Refraktionskorrektur erforderlich ist, als auch in Fällen, bei denen der vorherige Eingriff nicht zu einem ordnungsgemäßen Abschluss gebracht wurde, also abgebrochen wurde, vorgenommen werden. Insbesondere für letztere Fälle gab es im Stand der Technik bislang überhaupt kein brauchbares Mittel, um noch durch ein augenchirurgisches Verfahren eine Refraktionskorrektur zu erreichen.

Eine Nachbehandlung erfolgt vorteilhafterweise derart, dass die von einer Planungseinrichtung bzw. im Planungsverfahren festgelegte Hornhaut-Schnittfläche bzw. die in der Behandlungsvorrichtung bzw. beim Behandlungsverfahren erzeugte Hornhaut-Schnittfläche die voroperativen Schnitte nicht schneidet. Dies hat den Vorteil, dass nicht unerwünscht Volumina in der Hornhaut isoliert werden, die möglicherweise bei der Entnahme des eigentlich angestrebten Volumens mit aus der Hornhaut entfernt werden und zu einer unvorhersehbaren Veränderung der Hornhautoberfläche führen. Weiter ist auch vermieden, dass beim Abklappen der während der Nachbehandlung isolierten Lamelle diese unerwünscht geschwächt ist oder ungewollt weitere Teile der Augenhornhaut aufgrund eines nicht ausreichend berücksichtigten voroperativen Schnittes auf nicht vorherbestimmte Art und Weise abklappen.

Solche negativen Ereignisse während der Nachbehandlung können besonders zuverlässig vermieden werden, wenn die Berechnungsmittel der Planungseinrichtung bzw. das entsprechende Planungsverfahren die Schnittfläche in der Augenhornhaut so festlegen, dass das zu entnehmende Hornhaut-Volumen vollständig posterior der erzeugten voroperative Schnitte liegt oder vollständig anterior der voroperativen Schnitte liegt oder die erzeugten voroperativen Schnitte einschließt.

Die erste oder zweite Variante bieten sich insbesondere für Fälle an, in denen der vorherige Eingriff planmäßig ablief, jedoch noch eine Restfehlsichtigkeit zu korrigieren ist. Die dritte Variante bietet sich für Fälle an, in denen der vorherige Eingriff - aus welchen Gründen auch immer - abgebrochen wurde, da die dann zurückgebliebenen, möglicherweise hinsichtlich ihrer Lage nicht mit letzter Genauigkeit anzugebenden, voroperativen Schnitte mit der Entnahme des in der Nachbehandlung isolierten Hornhaut-Volumens aus der Hornhaut entfernt werden.

Ist hingegen die Lage der voroperativen Schnitte mit hinreichender Genauigkeit bestimmt bzw. bestimmbar, kann in einer Alternative der Erfindung die Hornhaut-Schnittfläche als Fortsetzung der bestehenden voroperativen Schnitte festgelegt werden.

Der Planung der Hornhaut-Schnittfläche kommt im Falle einer Nachbehandlung natürlich besondere Bedeutung zu, da die bestehenden voroperativen Schnitte zu berücksichtigen sind. Dies ist dem Operateur erleichtert, wenn eine Anzeigeeinrichtung zur visuellen Darstellung der Hornhaut und der bestehenden voroperativen Schnitte, vorzugsweise überlagerte Darstellung, vorgesehen ist.

Es ist ein besonderer Vorteil, dass zur Planung und Ausführung der Nachbehandlung besondere Daten verwendet werden können. Dabei kann es sich um im Gerät abgelegte Daten des vorherigen Eingriffes, nach dem vorherigen Eingriff und vor der Nachbehandlung erhobene diagnostische Daten des zu behandelnden Auges oder intraoperativ, d.h. während der Nachbehandlung, erhobene Daten des zu behandelnden Auges handeln. Solche Hornhaut-Daten werden aus einer Vermessung des Auges erzeugt und der Planungseinrichtung zugeführt, wobei eine Messeinrichtung verwendet wird, welche optional eine oder mehrere der folgenden Einrichtungen aufweist: Autorefraktor, Refraktometer, Keratometer, Aberrometer, Wellenfrontvermessungseinrichtung, OCT, konfokale Hornhautmikroskopie, Scheimpflugkamera, Topographiemessung.

Findet eine Nachbehandlung wegen eines Restkorrekturbedarfes statt, d.h. wurde der vorherige Eingriff planmäßig abgeschlossen, ist eine besonders wichtige Angabe natürlich die zu korrigierende Fehlsichtigkeit und/oder eine Angabe über Dicke und/oder Durchmesser einer voroperativ erzeugten, abklappbaren Hornhaut-Lamelle.

Eine Nachbehandlung ist umso einfacher bzw. präziser möglich, je genauer die Kenntnisse über die voroperativ vorhandenen Schnitte sind. Es ist deshalb ganz grundsätzlich und unabhängig von der Realisierung der Nachbehandlung von Vorteil, dass die laserchirurgische Behandlungsvorrichtung zur refraktiven Augenchirurgie eine Einrichtung aufweist, welche während eines Eingriffs den Fortschritt der erzeugten Schnitte protokolliert.

Arbeitet die Behandlungsvorrichtung mit gepulster Laserstrahlung, umfasst die Protokollierung vorzugsweise Lage und Energie jedes in die Hornhaut fokussierten Laserstrahlungspulses. Auch die relative Lage von Hornhaut (bzw. Auge) und Gerät wird protokolliert. Eine solche Protokollierung ist im Stand der Technik nicht bekannt und geht auch weit über das übliche Maß hinaus, das Angaben über den Patienten, den Refraktionskorrekturbedarf und bestenfalls noch die verwendete Schnittflächengeometrie umfasst. Der doch beträchtliche Datenspeicheraufwand erweist sich allerdings als vorteilhaft, wenn eine Nachbehandlung erforderlich ist, insbesondere bei nicht vollständig ausgeführtem vorherigen Eingriff. Dann ist eine Fortsetzung der abgebrochenen Schnittflächenerzeugung einfach und präzise möglich.

Bei der Nachbehandlung werden optional die festgelegten bzw. verwendeten Schnittflächen geometrisch so angeordnet, dass entweder überhaupt kein Schnitt mit dem voroperativen, bereits bestehenden Schnitt gegeben ist, oder dass dieser Schnitt geeignet ergänzt bzw. fortgesetzt wird.

Mehrere Fälle sind zu unterscheiden. Im Falle einer refraktiven Nachkorrektur, bei der das vorherige Verfahren ordnungsgemäß zum Abschluss gebracht wird, können die weiteren Schnitte so angeordnet werden, dass sie in einer geringeren Tiefe unterhalb der HornhautOberfläche liegen, als der voroperativ bestehende Schnitt, welcher zur Abklappung der Hornhaut-Lamelle erzeugt wurde. Alternativ ist es möglich, die Hornhaut-Schnittfläche in einer größeren Tiefe als den voroperativ bestehenden Schnitt anzuordnen. Das nun zu entnehmende Lentikel wird dann aus einem Hornhautbereich entnommen, der unterhalb der voroperativ erzeugten Hornhaut-Lamelle liegt. Im zuvor genannten Fall lag dagegen das Lentikel in der Hornhaut-Lamelle.

Diese Varianten gehen, ebenso wie die Variante, bei der das Hornhaut-Volumen den voroperativ bestehenden Schnitt umgibt, davon aus, dass das zu entnehmende HornhautVolumen, welches regelmäßig als Lentikel ausgebildet ist, ohne Mitwirkung der voroperativen Schnitte erzeugt wird, z.B. durch einen eigenständigen Flapschnitt sowie einen eigenständigen Lentikel-Schnitt.

Alternativ dazu ist es möglich, den bereits bestehenden voroperativen Schnitt zu verwenden und die Hornhaut-Schnittfläche so festzulegen, dass sie diesen Schnitt bei der Isolierung des zur Nachbehandlung zu entnehmenden Hornhaut-Volumens ergänzt bzw. ausnutzt. Das Lentikel wird dann durch den voroperativ bestehenden Schnitt sowie die festgelegte Hornhaut-Schnittfläche begrenzt. Damit erreicht man eine schnellere Schnittflächenerzeugung, bedarf allerdings genauer Kenntnisse über den voroperativen Schnitt. Natürlich kann die Ergänzung oberhalb des voroperativen Schnittes, d.h. durch die Hornhaut-Lamelle, oder unterhalb des voroperativen Schnittes, d.h. in Richtung Hornhaut-Innenfläche, hin vorgenommen werden.

Im Falle einer Nachbehandlung, der ein nicht ordnungsgemäß abgeschlossener vorheriger Eingriff voranging, ergibt sich stets das Problem, dass je nach Erzeugung des voroperativen Schnittes möglicherweise nur Schnittflächen vorhanden sind, die unvollständig sind. Beispielsweise kann ein Lentikel-Schnitt, welcher das Hornhaut-Volumen, das im vorherigen Eingriff entnommen werden sollte, posterior begrenzen sollte, vollständig oder teilweise ausgeführt sein. Möglicherweise besteht auch noch ein partieller oder gar fast vollständiger Flap-Schnitt, welcher das Lentikel anterior begrenzen sollte. Es kann sogar ein Fall vorliegen, in dem der Randschnitt, welcher dann das Abklappen der Hornhaut-Lamelle ermöglicht, der einzige Schnitt ist, der noch nicht vollständig ausgeführt ist. In jedem Fall bietet es sich bei genauer Kenntnis der erzeugten voroperativen Schnitte an, diese Schnitte fortzusetzen. Die Fortsetzung kann dabei auch beinhalten, dass die Schnittfläche den bereits erzeugten voroperativen Schnitt teilweise enthält, d.h. mit der Erzeugung der Schnittfläche in der Nachbehandlung in einem Bereich begonnen wird, in dem ein voroperativer Schnitt schon erwartet wird. Ein Überlapp sichert eine durchgängige Gewebetrennung im Zusammenwirken von voroperativem Schnitt und in der Nachbehandlung erzeugter Schnittfläche. Möchte man die voroperativen Schnitte nicht fortsetzen, beispielsweise weil ihre Position nicht hinreichend genau ist oder weil die Qualität dieser Schnitte nicht zufriedenstellend ist, ist es zweckmäßig, die Schnittfläche zur Isolierung des zu entnehmenden Hornhaut-Volumens so festzulegen, dass sie entweder durchgängig anterior der voroperativen Schnitte, durchgängig posterior der voroperativen Schnitte oder die voroperativen Schnitte im zu entnehmendes Hornhaut-Volumen einschließend ausgebildet sind.

Wie die Ausführungen zur Nachbehandlung erkennen lassen, kommt für die möglichst exakte Positionierung der Hornhaut-Schnittflächen mit der Positionsbestimmung des voroperativen Schnittes eine hohe Bedeutung zu. Hierbei sind folgende Varianten möglich:

1. Die Position des voroperativen Schnittes wird durch diagnostische Methoden vor der Durchführung der Nachbehandlung gemessen. Die diagnostischen Methoden können dabei unter anderem eine konfokale Hornhautmikroskopie, eine optische Kohärenztomographie oder die Verwendung einer Spaltlampe mit angeschlossener Messkamera umfassen.

2. Aus geräteinternen Daten des Gerätes, mit dem der vorherige Eingriff durchgeführt wurde, können Positionsdaten der voroperativen Schnitte abgeleitet werden. Dies kann umfassen, dass die Eingabedaten, mit welchen der vorherige Eingriff geplant wurde, verwendet werden und/oder Daten, welche während der Durchführung des vorherigen Eingriffes gewonnen wurden, für die Planung der Nachbehandlung verwendet werden. Beispiele für Daten, welche während der Durchführung des vorherigen Eingriffes gewonnen werden können, sind: Daten aus einer intraoperativen Messung der Reststromadicke, Daten über den Zeitpunkt des Abbruchs des Eingriffes und/oder Daten über die reale Position der im vorherigen Eingriff erzeugten Schnitte.

3. Positionsdaten über die voroperativ erzeugten und damit bei der Nachbehandlung vorhandenen Schnitte können auch durch ein Messsystem ermittelt werden, welches in der Behandlungsvorrichtung der Nachbehandlung vorgesehen ist, d.h. die Positionsdaten über die voroperativ vorhandenen Schnitte werden intraoperativ bestimmt. Ein solches Messsystem kann einen konfokalen Sensor oder auch optische Kohärenztomographie-Prinzipien verwenden. Möglich ist es auch, mittels der Behandlungsvorrichtung einen Probeschnitt auszuführen und aus den Daten einer Beobachtungskamera die Position des voroperativ vorhandenen Schnittes abzuleiten.

Um die Position des voroperativ vorhandenen Schnittes beschreiben zu können, ist im allgemeinen Falle die vollständige Angabe der Funktion $z(x,y)$ für alle Koordinatenpaare $(x,y)$ der Fläche des Schnittes erforderlich. Jedoch ist deutliche Reduktion der Parameter möglich, z.B. bei einem kreisrunden Flap homogener Dicke. In diesem Fall genügt die Bestimmung von Flapdicke, Flapdurchmesser und Position der Flapmitte.

Während der Durchführung eines laserchirurgischen Eingriffs, der mit gepulster Laserstrahlung erfolgt, folgt die Position des Laserfokus zum Zweck der Erzeugung einer zweidimensionalen Schnittfläche $S := R^2 \to R^3$ einer im Voraus berechneten Trajektorie $\vec{s}(t):=R^1 \to R^3$ mit $t \in I = 0, T \subset R$. Dabei sei $R$ die Menge der reellen Zahlen und $R^n$ der euklidische Verktorraum n-ter Dimension.

Einzelne Teilschnittflächen $S_j \subset S$ werden dabei sequenziell durch $\vec{s}(t)$ repräsentiert, d.h. es existiert zu jeder Teilschnittfläche $S_j$ genau ein Intervall $I_j = [t_1,t_2]_j$ aus dem die Trajektorie s(t) aus $S_j$ stammt. Durch die strikte Sequenzierung der Schnittflächen $S_j$. in der Trajektorie $\vec{s}$(t) sind die einzelnen Intervalle disjunkt, d.h. es gilt

$$\exists! I_j \subset I \left| \forall t \in I_j \right| \vec{s}(t) \in S_j \wedge \forall i \neq j \left| I_i \cap I_j = \varnothing \right. .$$

Für eine bestimmte Lasertherapie lässt sich die entsprechende Trajektorie $\vec{s}(t)$ zusammen mit dem zugehörigen Satz von disjunkten Intervallen $I_j$ berechnen. Während der Lasertherapie durchläuft der Parameter $t$ das Intervall $I =$ [0,$T$]. Die Therapie ist abgeschlossen, wenn $t=T$ oder mindestens $\forall k \in \bigcup_j I_j \left| t \geq k \right.$ ist.

Wird die Therapie bei $t=t_{int}$ abgebrochen, so kann mit Hilfe der Intervalle $I_j$ eindeutig festgestellt werden, bei welcher Schnittfläche der Abbruch stattgefunden hat, bzw. in wieweit diese Schnittfläche fertig gestellt worden ist. Eine Teilschnittfläche $S_j$ ist fertiggestellt, wenn gilt: $\forall k \in I_j | k \leq t_{int}$. Um also den Ort des Abbruchs zu bestimmen, genügt es, den Zeitpunkt des Abbruchs zu erfassen. Hierbei kann der Zeitpunkt mit einer gewissen Unschärfe erfasst werden, also beispielsweise etwa im Zeittakt von 1% von T.

Die Erfassung des Abbruchzeitpunktes bringt eine Vereinfachung und Erhöhung der Sicherheit der Protokollierung. Es ist deshalb vorteilhaft, die Protokollierung dahingehend zu vereinfachen, dass nicht die Lage eines jeden, in die Hornhaut abgegebenen Laserstrahlungspulses, protokolliert wird, sondern lediglich Parameter der Laserstrahlungspulsabgabe (z.B. Frequenz der Pulse), der Ablenkung des Fokus (z.B. Ablenkgeschwindigkeit) sowie die genaue Zeitangabe eines eventuellen Eingriffabbruches und Schnittgeometrieangaben.

Wie die voroperativen Schnitte erzeugt wurden ist für die Erfindung nicht ausschlaggebend. Sie können also im Prinzip auch mit einem mechanischen Mikrokeraton o. ä. erzeugt worden sein.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:

Fig. 1    eine schematische Darstellung einer Behandlungsvorrichtung mit einer Planungseinrichtung für eine Nachbehandlung bei augenchirurgischer Refraktionskorrektur,

Fig. 2    eine schematische Darstellung der Wirkung der Laserstrahlung, die in der Behandlungsvorrichtung der Fig. 1 verwendet wird,

Fig. 3    eine weitere Schemadarstellung des Behandlungsgerätes der Fig. 1 hinsichtlich der Einbringung der Laserstrahlung,

Fig. 4    eine schematische Schnittdarstellung durch die Augenhornhaut zur Veranschaulichung der Entnahme des Hornhaut-Volumens im Zusammenhang mit der augenchirurgischen Refraktionskorrektur,

Fig. 5    eine Schemadarstellung hinsichtlich des Aufbaus des Behandlungsgerätes der Fig. 1 mit besonderem Bezug auf die dort vorhandene Planungseinrichtung,

Fig. 6    eine schematische Schnittdarstellungen durch die Augenhornhaut im Zusammenhang mit der augenchirurgischen Refraktionskorrektur bei einer Nachbehandlung zur Korrektur einer Restfehlsichtigkeit,

Fig. 7    eine schematische Schnittdarstellung durch die Augenhornhaut im Zusammenhang mit der augenchirurgischen Refraktionskorrektur bei einer Nachbehandlung zur Fortsetzung eines abgebrochenen Vor-Eingriffes,

Fig. 8      eine weitere schematische Schnittdarstellung durch die Augenhornhaut im Zusammenhang mit der augenchirurgischen Refraktionskorrektur bei einer Nachbehandlung zur Fortsetzung eines abgebrochenen Vor-Eingriffes unter Fortsetzung der bestehenden Schnittfläche und

Fig. 9      eine weitere schematische Schnittdarstellung durch die Augenhornhaut im Zusammenhang mit der augenchirurgischen Refraktionskorrektur bei einer Nachbehandlung zur Korrektur einer Restfehlsichtigkeit unter Verwendung der bestehenden Schnittfläche.

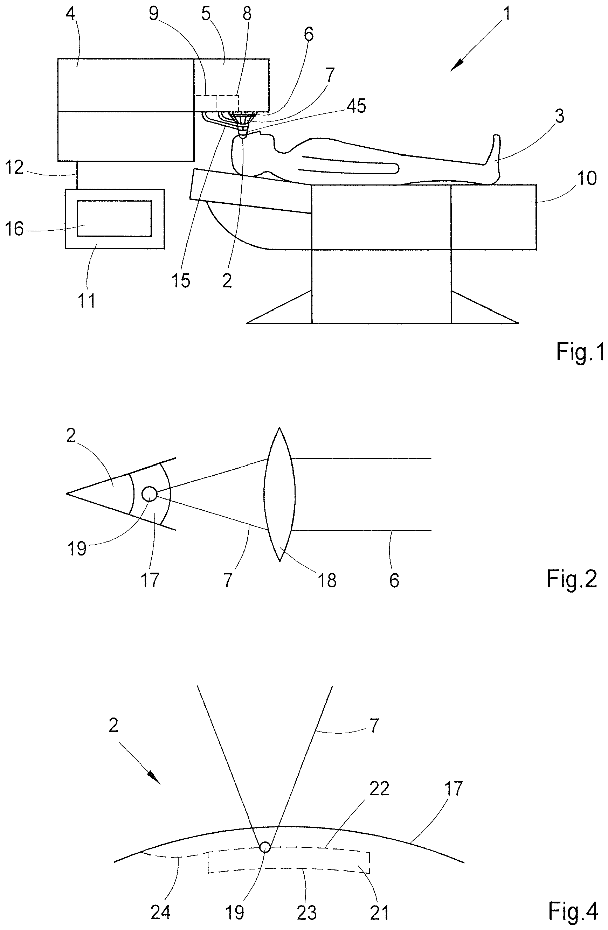

Eine Behandlungsvorrichtung für die augenchirurgische Refraktionskorrektur ist in Fig. 1 dargestellt und mit dem allgemeinen Bezugszeichen 1 versehen. Die Behandlungsvorrichtung 1 ist für eine nachbehandelnde Refraktionskorrektur am einem Auge 2 eines Patienten 3 ausgebildet. Dazu weist die Behandlungsvorrichtung 1 eine Lasereinrichtung 4 auf, die aus einer Laserquelle 5 einen Laserstrahl 6 abgibt, welcher als fokussierter Strahl 7 in das Auge 2 bzw. die Augenhornhaut gerichtet wird. Vorzugsweise ist der Laserstrahl 6 ein gepulster Laserstrahl mit einer Wellenlänge zwischen 400 Nanometer und 10 Mikrometer. Weiter liegt die Pulslänge des Laserstrahls 6 im Bereich zwischen 1 Femtosekunde und 10 Pikosekunden, wobei Pulswiederholraten von 1 bis 1000 Kilohertz und Pulsenergien zwischen 0,01 Mikrojoule und 0,01 Millijoule möglich sind. Die Behandlungsvorrichtung 1 erzeugt somit in der Hornhaut des Auges 2 durch Ablenkung der gepulsten Laserstrahlung eine Schnittfläche. In der Lasereinrichtung 4 bzw. deren Laserquelle 5 ist deshalb dazu noch ein Scanner 8 sowie ein Strahlungsintensitätsmodulator 9 vorgesehen.

Der Patient 3 befindet sich auf einer Liege 10, die in drei Raumrichtungen verstellbar ist, um das Auge 2 passend zum Einfall des Laserstrahls 6 auszurichten. In bevorzugter Bauweise ist die Liege 10 motorisch verstellbar.

Die Ansteuerung kann insbesondere durch ein Steuergerät 11 erfolgen, das grundsätzlich den Betrieb der Behandlungsvorrichtung 1 steuert und dazu über geeignete Datenverbindungen, beispielsweise Verbindungsleitungen 12 mit der Behandlungsvorrichtung verbunden ist. Natürlich kann diese Kommunikation auch über andere Wege, beispielsweise Lichtleiter oder per Funk geschehen. Das Steuergerät 11 nimmt die entsprechenden Einstellungen, Zeitsteuerung an der Behandlungsvorrichtung 1, insbesondere der Lasereinrichtung 4 vor und bewerkstelligt damit entsprechende Funktionen der Behandlungsvorrichtung 1.

Die Behandlungsvorrichtung 1 weist weiter noch eine Fixiereinrichtung 15 auf, welche die Hornhaut des Auges 2 gegenüber der Lasereinrichtung 4 lagefixiert. Diese Fixiereinrichtung 15 kann dabei ein bekanntes Kontaktglas 45 umfassen, an das die Augenhornhaut durch Unterdruck angelegt wird und das der Augenhornhaut eine gewünschte geometrische Form verleiht. Solche Kontaktgläser sind dem Fachmann aus dem Stand der Technik bekannt, beispielsweise aus der DE 102005040338 A1. Der Offenbarungsgehalt dieser Druckschrift wird, soweit die Beschreibung einer Bauform des für die Behandlungsvorrichtung 1 möglichen Kontaktglases 45 betroffen ist, hier vollumfänglich einbezogen.

Das Steuergerät 11 der Behandlungsvorrichtung 1 weist weiter noch eine Planungseinrichtung 16 auf, die später noch näher erläutert werden wird.

Fig. 2 zeigt schematisch die Wirkungsweise des einfallenden Laserstrahls 6. Der Laserstrahl 6 wird fokussiert und fällt als der fokussierte Laserstrahl 7 in die Hornhaut 17 des Auges 2. Zur Fokussierung ist eine schematisch eingezeichnete Optik 18 vorgesehen. Sie bewirkt in der Hornhaut 17 einen Fokus, in dem die Laserstrahlungsenergiedichte so hoch ist, dass in Kombination mit der Pulslänge der gepulsten Laserstrahlung 6 ein nicht-linearer Effekt in der Hornhaut 17 auftritt. Beispielsweise kann jeder Puls der gepulsten Laserstrahlung 6 im Fokus 19 einen optischen Durchbruch in der Augenhornhaut 17 erzeugen, welche wiederum eine in Fig. 2 nur schematisch angedeutete Plasmablase initiiert. Bei Entstehung der Plasmablase umfasst die Gewebsschichttrennung ein größeres Gebiet als den Fokus 19, obwohl die Bedingungen zur Erzeugung des optischen Durchbruches nur im Fokus 19 erreicht werden. Damit von jedem Laserpuls ein optischer Durchbruch erzeugt wird, muß die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt. Alternativ kann ein gewebetrennender Effekt auch durch gepulste Laserstrahlung erreicht werden, indem mehrere Laserstrahlungspulse in einem Bereich abgegeben werden, wobei sich die Fokus-Spots überlappen. Es wirken dann mehrere Laserstrahlungspulse zusammen, um einen gewebetrennenden Effekt zu erreichen.

Die Art der Gewebetrennung, die die Behandlungsvorrichtung 1 einsetzt, ist jedoch für die nachfolgende Beschreibung nicht weiter relevant; wesentlich ist lediglich, dass eine Schnittflächenerzeugung in der Hornhaut 17 des Auges 2 stattfindet.

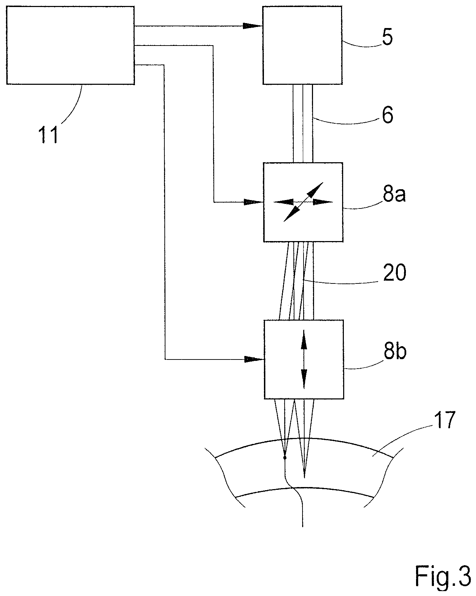

Um nun eine augenchirurgische Refraktionskorrektur auszuführen, wird mittels der Laserstrahlung 6 aus einem Gebiet innerhalb der Hornhaut 17 ein Hornhautvolumen entfernt, indem dort Gewebeschichten getrennt werden, die das Hornhaut-Volumen isolieren und dann dessen Entnahme ermöglichen. Zur Isolierung des zu entfernenden Hornhaut-Volumens wird z.B. im Falle der gepulst eingebrachten Laserstrahlung die Lage des Fokus 17 der fokussierten Laserstrahlung 7 in der Hornhaut 17 verstellt. Dies ist schematisch in Fig. 3 gezeigt. Die Brechungseigenschaften der Hornhaut 17 werden durch die Entnahme des Volumens gezielt verändert, um so die Refraktionskorrektur zu erreichen. Das Volumen ist deshalb meist linsenförmig und wird als Lentikel bezeichnet. Die Entfernung des Hornhaut-Volumens erfolgt hier als Nachbehandlung. Ihr ging entweder eine augenchirurgische Refraktionskorrektur voraus, die noch einen Restkorrekturbedarf hinterließ, oder gar eine während des Eingriffes abgebrochene augenchirurgische Refraktionskorrektur, bei der Schnittflächen nur unvollständig erzeugt wurden. Natürlich ist auch dann noch ein Korrekturbedarf gegeben.

In Fig. 3 sind die Elemente der Behandlungsvorrichtung 1 nur insoweit eingetragen, als sie zum Verständnis der Schnittflächenerzeugung erforderlich sind. Der Laserstrahl 6 wird, wie bereits erwähnt, in einem Fokus 19 in der Hornhaut 17 gebündelt, und die Lage des Fokus 19 in der Hornhaut wird verstellt, so dass zur Schnittflächenerzeugung an verschiedenen Stellen fokussierte Energie aus Laserstrahlungspulsen in das Gewebe der Hornhaut 17 eingetragen wird. Die Laserstrahlung 6 wird von der Laserquelle 5 vorzugsweise als gepulste Strahlung bereitgestellt. Der Scanner 8 ist in der Bauweise der Fig. 3 zweiteilig aufgebaut und besteht aus einem xy-Scanner 8a, der in einer Variante durch zwei im Wesentlichen orthogonal ablenkende Galvanometerspiegel realisiert ist. Der Scanner 8a lenkt den von der Laserquelle 5 kommenden Laserstrahl 6 zweidimensional ab, so dass nach dem Scanner 8 ein abgelenkter Laserstrahl 20 vorliegt. Der Scanner 8a bewirkt somit eine Verstellung der Lage des Fokus 19 im Wesentlichen senkrecht zur Haupteinfallsrichtung des Laserstrahls 6 in der Hornhaut 17. Zur Verstellung der Tiefenlage ist neben dem xy-Scanner 8a im Scanner 8 noch ein z-Scanner 8b vorgesehen, der beispielsweise als verstellbares Teleskop ausgebildet ist. Der z-Scanner 8b sorgt dafür, dass die z-Position der Lage des Fokus 19, d.h. dessen Position auf der optischen Achse des Einfalls verändert wird. Der z-Scanner 8b kann dem xy-Scanner 8a nach- oder vorgeordnet sein.

Für das Funktionsprinzip der Behandlungsvorrichtung 1 ist die Zuordnung der einzelnen Koordinaten zu dem Raumrichtungen nicht wesentlich, genau so wenig, dass der Scanner 8a um zueinander rechtwinklige Achsen ablenkt. Vielmehr kann jeder Scanner verwendet werden, der in der Lage ist, den Fokus 19 in einer Ebene zu verstellen, in der die Einfallsachse der optischen Strahlung nicht liegt. Weiter können auch beliebige nicht-kartesische Koordinatensysteme zur Ablenkung bzw. Steuerung der Lage des Fokus 19 verwendet werden. Beispiele dafür sind Kugelkoordinaten oder zylindrische Koordinaten.

Die Steuerung der Lage des Fokus 19 erfolgt mittels der Scanner 8a, 8b unter Ansteuerung durch das Steuergerät 11, das entsprechende Einstellungen an der Laserquelle 5, dem (in Fig. 3 nicht gezeigten) Modulator 9 sowie dem Scanner 8 vornimmt. Das Steuergerät 11 sorgt für einen geeigneten Betrieb der Laserquelle 5 sowie die hier exemplarisch geschilderte dreidimensionale Fokusverstellung, so dass letztendlich eine Schnittfläche ausgebildet wird, die ein bestimmtes Hornhaut-Volumen isoliert, das zur Refraktionskorrektur entfernt werden soll.

Die Steuereinrichtung 11 arbeitet nach vorgegebenen Steuerdaten, welche beispielsweise bei der hier lediglich exemplarisch geschilderten Lasereinrichtung 4 als Zielpunkte für die Fokusverstellung vorgegeben sind. Die Steuerdaten sind in der Regel in einem Steuerdatensatz zusammengefasst. Dies macht geometrische Vorgaben für die auszubildende Schnittfläche, beispielsweise die Koordinaten der Zielpunkte als Muster. Der Steuerdatensatz enthält dann in dieser Ausführungsform auch konkrete Stellwerte für den Fokuslagenverstellmechanismus, z.B. für den Scanner 8.

Die Erzeugung der Schnittfläche mit der Behandlungsvorrichtung 1 ist exemplarisch in Fig. 4 gezeigt. Ein Hornhaut-Volumen 21 in der Hornhaut 17 wird durch Verstellung des Fokus 19, in dem der fokussierte Strahl 7 gebündelt ist, isoliert. Dazu werden Schnittflächen ausgebildet, die hier exemplarisch als anteriore Flap-Schnittfläche 22 sowie als posteriore Lentikel-Schnittfläche 23 ausgebildet sind. Diese Begriffe sind hier lediglich exemplarisch zu verstehen und sollen den Bezug auf das herkömmliche Lasik- oder Flex-Verfahren herstellen, für das die Behandlungsvorrichtung 1, wie bereits geschildert, ausgebildet ist. Wesentlich ist hier lediglich, dass die Schnittflächen 22 und 23 sowie nicht weiter bezeichnete Randschnitte, welche die Schnittflächen 22 und 23 an deren Rändern zusammenführen, das Hornhaut-Volumen 21 isolieren. Durch einen Öffnungsschnitt 24 kann weiter eine das Hornhaut-Volumen 21 anterior begrenzende Hornhaut-Lamelle abgeklappt werden, so dass das Hornhaut-Volumen 21 entnehmbar ist.

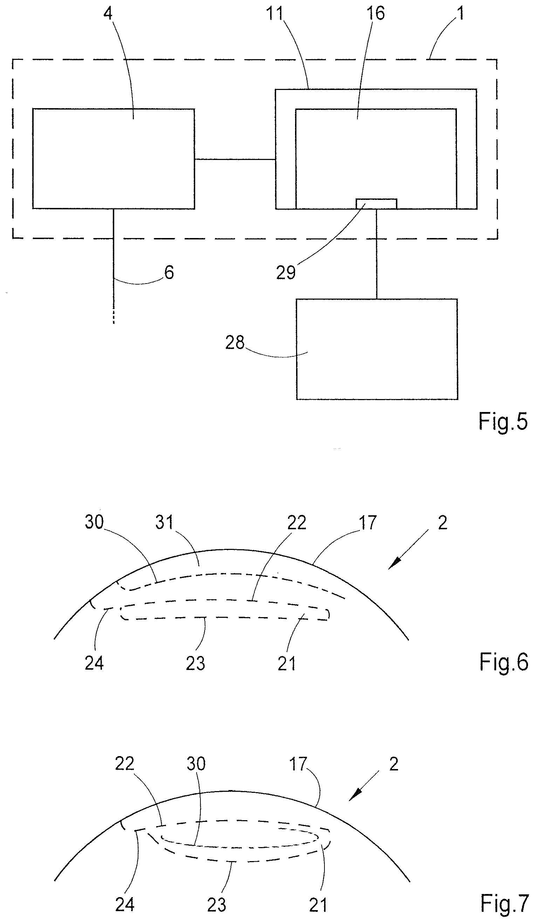

Fig. 5 zeigt schematisch die Behandlungsvorrichtung 1, und anhand ihr soll die Bedeutung der Planungseinrichtung 16 näher erläutert werden. Die Behandlungsvorrichtung 1 weist in dieser Variante mindestens zwei Einrichtungen oder Module auf. Die bereits geschilderte Lasereinrichtung 4 gibt den Laserstrahl 6 auf das Auge 2 ab. Der Betrieb der Lasereinrichtung 4 erfolgt dabei, wie bereits geschildert, voll automatisch durch das Steuergerät 11, d.h. die Lasereinrichtung 4 startet auf ein entsprechendes Startsignal hin die Erzeugung und Ablenkung des Laserstrahls 6 und erzeugt dabei Schnittflächen, die auf die beschriebene Art und Weise aufgebaut sind, um das Hornhaut-Volumen 21 zu entnehmen. Die für den Betrieb erforderlichen Steuersignale empfängt die Lasereinrichtung 5 vom Steuergerät 11, dem zuvor entsprechende Steuerdaten bereitgestellt wurden. Dies erfolgt mittels der Planungseinrichtung 16, die in Fig. 6 lediglich exemplarisch als Bestandteil des Steuergeräts 11 gezeigt ist. Natürlich kann die Planungseinrichtung 16 auch eigenständig ausgebildet sein und drahtgebunden oder drahtlos mit der Steuereinrichtung 11 kommunizieren. Wesentlich ist dann lediglich, dass ein entsprechender Datenübertragungskanal zwischen der Planungseinrichtung 16 und dem Steuergerät 11 vorgesehen ist.

Die Planungseinrichtung 16 erzeugt einen Steuerdatensatz, der dem Steuergerät 11 zur Ausführung der augenchirurgischen Refraktionskorrektur zur Verfügung gestellt wird. Dabei verwendet die Planungseinrichtung Messdaten über die Hornhaut des Auges. Diese Daten stammen in der hier beschriebenen Ausführungsform aus einer Messeinrichtung 28, die das Auge 2 des Patienten 2 zuvor vermessen hat. Eine ähnliche Messeinrichtung 28 kann auch für das Transplantationsmaterial vorgesehen werden. Natürlich kann die Messeinrichtung 28 auf beliebige Art und Weise ausgebildet sein und die entsprechenden Daten an die Schnittstelle 29 der Planungseinrichtung 16 übermitteln.

Die Daten über das Transplantationsmaterial können auch aus anderen Quellen stammen und müssen nicht zuvor direkt durch eine Messung erzeugt werden.

Die Planungseinrichtung unterstützt nun den Bediener der Behandlungsvorrichtung 1 bei der Festlegung der Schnittfläche zur Isolierung des Hornhaut-Volumens 21. Dies kann bis zu einer voll automatischen Festlegung der Schnittflächen gehen, die beispielsweise dadurch bewirkt werden kann, dass die Planungseinrichtung 16 aus den Messdaten das zu entnehmende Hornhaut-Volumen 21 ermittelt, dessen Begrenzungsflächen als Schnittflächen definiert und daraus entsprechende Steuerdaten für das Steuergerät 11 erzeugt. Am anderen Ende des Automatisierungsgrades kann die Planungseinrichtung 16 Eingabemöglichkeiten vorsehen, an denen ein Benutzer die Schnittflächen in Form von geometrischen Parametern etc. eingibt. Zwischenstufen sehen Vorschläge für die Schnittflächen vor, welche die Planungseinrichtung 16 automatisch generiert und die von einem Bearbeiter dann modifizierbar sind. Grundsätzlich können all diejenigen Konzepte, die im vorstehend allgemeineren Beschreibungsteil bereits erläutert wurden, hier in der Planungseinrichtung 16 zur Anwendung kommen.

Um eine Nachbehandlung durchzuführen, erzeugt die Planungseinrichtung 16 Steuerdaten für die Schnittflächenerzeugung, die dann in der Behandlungsvorrichtung 1 verwendet werden. Figur 6 zeigt exemplarisch die mögliche Lage der Schnittflächen, wobei Schnittflächen, die denen der Figur 4 entsprechen, mit denselben Bezugszeichen versehen sind. Der wesentliche Unterschied zur Situation der Figur 4 liegt nun darin, dass in der Hornhaut 17 bereits ein älterer Schnitt 30 vorhanden ist, der von einem vorherigen Eingriff stammt, beispielsweise einem Eingriff nach dem Flex-Verfahren. Der ältere Schnitt 30 ist in Figur 6 wie in den nachfolgenden Figuren mit einer strichpunktierten Linie eingezeichnet. Zur Unterscheidung vom älteren Schnitt 30 sind die für die Nachbehandlung vorgesehenen Schnittflächen gestrichelt gezeichnet.

Wie Figur 6 zeigt, werden die Steuerdaten so festgelegt, dass die NachbehandlungsSchnittflächen zur Behebung des Restkorrekturbedarfes sämtlich unterhalb des älteren Schnittes 30 liegen. Es wird also bezogen auf den älteren Schnitt 30 posterior das zu entnehmende Hornhaut-Volumen 21 z.B. durch einen Lentikel-Schnitt 23 und einem Flap-Schnitt 22 samt seitlichem Öffnungsschnitt 24 erzeugt. Eine unerwünschte Interferenz mit dem älteren Schnitt 30 ist damit vermieden.

In einer (nicht gezeigten) Abwandlung der Schnittflächen der Figur 6 können die für die Nachbehandlung vorgesehenen bzw. verwendeten Schnittflächen auch sämtlich in der Hornhaut-Lamelle 31 liegen, die zwischen dem älteren Schnitt 30 und der Vorderfläche der Hornhaut 17 entstanden ist.

Figur 7 zeigt eine weitere Variante, die sich insbesondere dann anbietet, wenn der Grad der Ausbildung der älteren Schnitte nicht hinreichend genau bekannt ist. Die Nachbehandlungs-Schnittfläche, z.B. mit Lentikel-Schnitt 23 und Flap-Schnitt 22, ist nun so definiert, dass die älteren Schnitte 30 (wiederum strichpunktiert gezeichnet) vollständig innerhalb des Hornhaut-Volumens 21, welches zur Korrektur entnommen wird, liegen. Dieses Vorgehen hat den Vorteil, dass die Zahl der Grenzflächen in der Augenhornhaut, die nach dem Eingriff verbleibt, gering ist.

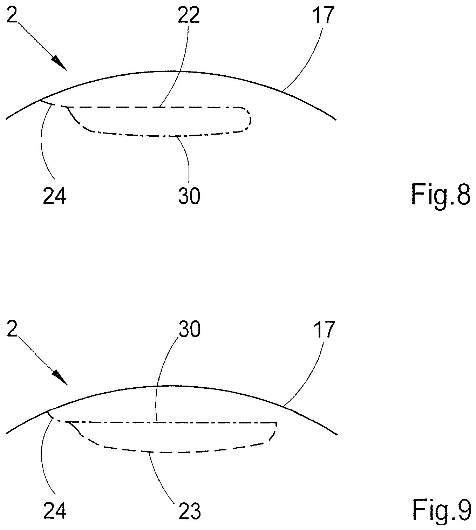

Figur 8 zeigt eine Möglichkeit, die sich insbesondere dann anbietet, wenn über die Lage der älteren Schnitte besonders gute Kenntnis besteht. Die Nachbehandlungsschnittflächen sind nun als Fortsetzung des älteren Schnittes 30 ausgebildet. Dies bietet sich natürlich dann an, wenn der vorherige Eingriff unerwünscht abgebrochen wurde.

Eine weitere Methode, ältere Schnitte zu verwenden, zeigt Figur 9, bei der das zu isolierende Hornhaut-Volumen 21 sowohl durch ältere Schnitte 30 als auch durch in der Nachbehandlung erfolgte Schnittflächen definiert ist. Exemplarisch ist hier der Einsatz des älteren Schnittes 30 als Flap-Schnitt gezeigt, der mit einem in der Nachbehandlung vorgesehenen Lentikel-Schnitt 23 ergänzt wird. Dies ist natürlich exemplarisch zu verstehen und es kann auch ein in die Lamelle 31 hineinverlaufender Schnitt zur Ergänzung in der Nachbehandlung verwendet werden.

Zusätzlich sei noch angemerkt, dass die Behandlungsvorrichtung 1 bzw. die Planungseinrichtung 16 natürlich auch die Durchführung des zuvor allgemein erläuterten Verfahrens konkret realisiert.

Eine weitere Ausführungsform der Planungseinrichtung besteht in Form eines Computerprogrammes bzw. eines entsprechenden Datenträgers mit einem Computerprogramm, der die Planungseinrichtung auf einem entsprechenden Computer realisiert, so dass die Eingabe der Messdaten bzw. Transplantationsmaterial-Daten über geeignete Datenübertragungsmittel an den Computer erfolgt und die Steuerdaten von diesem Computer an das Steuergerät 11 übertragen werden, wozu wiederum dem Fachmann bekannte Datenübertragungsmittel in Frage kommen.

**Patentansprüche**

1. Behandlungsvorrichtung zur Refraktion korrigierenden Augenchirurgie aufweisend

   - eine Lasereinrichtung (4), welche zur Abgabe eines Laserstrahls (6) auf das Auge (2) ausgebildet ist,
   - ein die Lasereinrichtung (4) gemäß Steuerdaten steuerndes Steuergerät (11) zur Erzeugung mindestens eine Schnittfläche (22, 23) in einer Augen-Hornhaut (17) mittels Laserstrahlung, und

- eine Planungseinrichtung (16) zum Erzeugen der Steuerdaten, wobei die Planungseinrichtung (16) eine Schnittstelle (29) zum Zuführen von Hornhaut-Daten aufweist und konfiguriert ist, die Hornhaut-Schnittfläche (22, 23) basierend auf den Hornhaut-Daten festzulegen und für die Hornhaut-Schnittfläche (22, 23) einen Steuerdatensatz mit den Steuerdaten zu erzeugen und dem Steuergerät (11) zur Verfügung zu stellen,
- wobei an der Schnittstelle (29) eine Messeinrichtung (28) angeschlossen ist, welcher die Hornhaut-Daten aus einer Vermessung des Auges (2) erzeugt und der Planungseinrichtung (16) zuführt,
- wobei die Behandlungsvorrichtung eine Einrichtung aufweist, welche während eines Eingriffs den Fortschritt der erzeugten Schnittfläche (22, 23) protokolliert.

2. Behandlungsvorrichtung nach Anspruch 1, **dadurch gekennzeichnet, dass** die Hornhaut-Daten Angaben über voroperative Schnitte (30) enthalten und die Planungseinrichtung (16) konfiguriert ist, die Hornhaut-Schnittfläche (22, 23) so festzulegen, dass sie die voroperativen Schnitte (30) nicht schneiden.

3. Behandlungsvorrichtung nach Anspruch 2, **dadurch gekennzeichnet, dass** die Planungseinrichtung (16) konfiguriert ist, die Schnittfläche in der Augenhornhaut so festzulegen, dass ein zu entnehmendes Hornhaut-Volumen (21)

   - vollständig posterior der erzeugten, voroperativen Schnitte (30) liegt oder
   - vollständig anterior der erzeugten, voroperativen Schnitte (30) liegt oder
   - die erzeugten, voroperativen Schnitte (30) einschließt.

4. Behandlungsvorrichtung nach Anspruch 2, **dadurch gekennzeichnet, dass** die Planungseinrichtung (16) konfiguriert ist, die Hornhaut-Schnittfläche als Fortsetzung der erzeugten, voroperativen Schnitte (30) festzulegen.

5. Behandlungsvorrichtung nach einem der obigen Ansprüche, **dadurch gekennzeichnet, dass** die Daten Angaben über eine zu korrigierende Fehlsichtigkeit und/oder Angaben über Dicke und/oder Durchmesser einer voroperativ erzeugten Hornhaut-Lamelle enthalten.

6. Behandlungsvorrichtung nach Anspruch 4, **dadurch gekennzeichnet, dass** die Angaben über voroperative Schnitte (30) während des Verlaufes des vorherigen Eingriffes oder unmittelbar im Anschluss daran erzeugte Daten umfassen, welche Lage und Form der Schnitte (30) wiedergeben.

7. Behandlungsvorrichtung nach Anspruch 1, wobei die Laserstrahlung gepulst ist.

8. Behandlungsvorrichtung nach Anspruch 7, wobei die Protokollierung Lage und Energie jedes in die Hornhaut fokussierten Laserstrahlungspulses und auch die relative Lage von Hornhaut oder Auge und Lasereinrichtung umfasst.

9. Vorrichtung nach Anspruch 1, 7 oder 8 **dadurch gekennzeichnet, dass** die Einrichtung zur Protokollierung Lage und Energie jedes in die Hornhaut fokussierten Laserstrahlungspulses protokolliert oder Parameter der Laserstrahlungspulsabgabe, einer Fokusablenkung sowie eine Zeitangabe über einen eventuellen Eingriffsabbruch und Schnittgeometrieangaben protokolliert.

**Claims**

1. A treatment apparatus for refraction-correcting ophthalmic surgery, said apparatus comprising

   - a laser device (4) configured to deliver a laser beam (6) to the eye (2),
   - a control device (11) for controlling the laser device (4) according to control data to generate at least one cut (22, 23) in the cornea (17) of the eye by laser radiation, and
   - a planning device (16) for generating the control data, wherein the planning device (16) comprising an interface (29) for receiving corneal data and being configured to define the corneal cut (22, 23) on basis of the corneal data and to generate a control dataset including the control data and to provide the control dataset to the control device (11),
   - wherein a measurement device (28) is connected to the interface (29), said measurement device (28) generating the corneal data from a measurement of the eye (2) and supplying them to the planning device (16),
   - wherein the treatment apparatus comprises a device which logs advance of the generated cut (22, 23) during surgery.

**2.** The treatment apparatus according to claim 1, **characterized in that** the corneal data include information on pre-operative cuts (30) which were generated in a previous ophthalmic surgery and the planning device (16) is configured to define the corneal cut (22, 23) such that it does not intersect the pre-operative cuts (30).

**3.** The treatment apparatus according to claim 2, **characterized in that** the planning device (16) is configured to define the cut in the cornea such that a corneal volume (21) to be removed

- is located completely posterior to the generated pre-operative cuts (30) or
- is located completely anterior to the generated pre-operative cuts (30) or
- confines the generated pre-operative cuts (30).

**4.** The treatment apparatus according to claim 2, **characterized in that** the planning device (16) is configured to define the corneal cut as a continuation of the generated pre-operative cuts (30).

**5.** The treatment apparatus according to any one of the above claims, **characterized in that** the data contain information on an eyesight defect to be corrected and/or information on thickness and/or diameter of a pre-operatively generated corneal lamella.

**6.** The treatment apparatus according to claim 4, **characterized in that** the information on pre-operative cuts (30) comprise data which were generated during the course of the previous operation or immediately thereafter and represent the positions and shapes of the cuts (30).

**7.** The treatment apparatus according to claim 1, wherein the laser radiation is pulsed.

**8.** The treatment apparatus according to claim 7, wherein the logging comprises position and energy of each laser beam pulse focused into the cornea and further the relative position between cornea or eye and laser device.

**9.** Apparatus according to claim 1, 7 or 8, **characterized in that** the device for logging logs position and energy of each laser beam pulsed focused into the cornea or parameter of laser pulse delivery, a focus shift as well as a time information on any surgery abort and cut geometry information.

**Revendications**

**1.** Dispositif de traitement pour la chirurgie oculaire destinée à corriger la réfraction, comportant

- un dispositif à laser (4), qui est conçu pour émettre un faisceau laser (6) vers l'œil (2),
- un appareil de commande (11) commandant le dispositif à laser (4) selon des données de commande pour produire au moins une surface de coupe (22, 23) dans une cornée oculaire (17) au moyen d'un rayonnement laser, et
- un dispositif de planification (16) destiné à produire les données de commande, dans lequel le dispositif de planification (16) présente une interface (29) destinée à délivrer des données de cornée et est configuré pour déterminer la surface de coupe (22, 23) de la cornée sur la base des données de cornée et pour produire un jeu de données de commande comprenant les données de commande pour la surface de coupe (22, 23) de la cornée et le mettre à disposition du dispositif de commande (11),
- dans lequel un dispositif de mesure (28) est raccordé à l'interface (29), lequel dispositif de mesure produit les données de cornée à partir d'une mesure de l'œil (2) et les délivre au dispositif de planification (16),
- dans lequel le dispositif de traitement comporte un dispositif qui, au cours d'une intervention, établit un protocole de la progression de la surface de coupe (22, 23) produite.

**2.** Dispositif de traitement selon la revendication 1, **caractérisé en ce que** les données de cornée contiennent des indications concernant des coupes préopératoires (30) et le dispositif de planification (16) est configuré pour déterminer la surface de coupe (22, 23) de la cornée de manière à ne pas couper les coupes préopératoires (30).

**3.** Dispositif de traitement selon la revendication 2, **caractérisé en ce que** le dispositif de planification (16) est configuré pour déterminer la surface de coupe dans la cornée oculaire de manière à ce qu'un volume de cornée (21) à éliminer

- se situe entièrement en position postérieure aux coupes préopératoires (30) produites ou

- se situe entièrement en position antérieure aux coupes préopératoires (30) produites ou
- inclut les coupes préopératoires (30) produites.

4.  Dispositif de traitement selon la revendication 2, **caractérisé en ce que** le dispositif de planification (16) est configuré pour déterminer la surface de coupe de la cornée sous la forme d'une poursuite des coupes préopératoires (30) produites.

5.  Dispositif de traitement selon l'une quelconque des revendications précédentes, **caractérisé en ce que** les données comprennent des indications concernant un défaut de vision à corriger et/ou des indications concernant une épaisseur et/ou un diamètre d'une lamelle de cornée produite de manière préopératoire.

6.  Dispositif de traitement selon la revendication 4, **caractérisé en ce que** les indications concernant les coupes préopératoires (30) comprennent des données produites au cours de l'intervention précédente ou directement à la suite de celle-ci, qui restituent la position et la forme des coupes (30).

7.  Dispositif de traitement selon la revendication 1, dans lequel le rayonnement laser est pulsé.

8.  Dispositif de traitement selon la revendication 7, dans lequel le protocole comprend la position et l'énergie de chaque impulsion de faisceau laser focalisée dans la cornée ainsi que la position relative de la cornée ou de l'œil et du dispositif à laser.

9.  Dispositif selon la revendication 1, 7 ou 8, **caractérisé en ce que** le dispositif de protocole enregistre la position et l'énergie de chaque impulsion de rayonnement laser focalisée dans la cornée ou enregistre des paramètres de l'émission d'impulsions de rayonnement laser, une déviation du foyer ainsi qu'une indication de temps concernant une éventuelle interruption de l'intervention et des indications de géométrie de coupe.

Fig.1

Fig.2

Fig.4

Fig.3

Fig.5

Fig.6

Fig.7

2   22   17

24   30

Fig.8

2   30   17

24   23

Fig.9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007012924 A1
- DE 102005040338 A1
- DE 69500997 T2